# EUROPÄISCHE PATENTSCHRIFT

(11) **EP 0 868 157 B1**
(45) Veröffentlichungstag und Bekanntmachung des Hinweises auf die Patenterteilung: **24.07.2002**
(21) Anmeldenummer: 96939050.9
(22) Anmeldetag: 15.11.1996
(51) Int. Cl.: A61F 2/30, A61L 27/00

(54) **IMPLANTAT ALS KNOCHENERSATZ**
IMPLANT AS BONE PROSTHESIS
IMPLANT SERVANT DE PROTHESE OSSEUSE

(30) Priorität: 22.11.1995 DE 19543530
(43) Veröffentlichungstag der Anmeldung: 07.10.1998
(73) Patentinhaber: ESKA Implants GmbH & Co., 23556 Lübeck (DE)
(72) Erfinder: GRUNDEI, Hans, D-23558 Lübeck (DE)
(74) Vertreter: Fuchs Mehler Weiss & Fritzsche
(86) Internationale Anmeldenummer: EP9605018
(87) Internationale Veröffentlichungsnummer: WO9718775

(56) Entgegenhaltungen:
- EP-A- 0 159 036
- EP-A- 0 399 163
- DE-C- 4 106 971
- DE-C- 4 208 247
- DE-C- 4 336 551
- FR-A- 2 356 465
- US-A- 4 550 448

## Beschreibung

Die Erfindung betrifft ein Implantat als Knochenersatz mit den Merkmalen des Oberbegriffs des Anspruchs 1, wie es beispielsweise nach Durchführung des Verfahrens gemäß DE-C 41 06 971 vorliegt.

Ein derartiges Implantat hat sich in den letzten Jahren hervorragend bewährt, insbesondere was das beabsichtigte Ein- und Durchwachsen der seine Oberfläche zumindest teilweise bedeckenden offenmaschigen, dreidimensionalen Struktur betrifft.

Das Einwachsverhalten des Implantates kann noch dadurch verbessert werden, daß gemäß der DE-C 42 08 247 mehrere diskrete Zonen mit unterschiedlicher Maschenweite auf der Oberfläche des Implantates erzeugt sind. Die Maschenweiten sind dabei der Zelligkeit und Porigkeit des Knochenmaterials, welches nach der Implantation mit der Oberflächenstruktur des Implantates in Verbindung steht, angepaßt, in dem Sinne, daß die jeweiligen Maschenweiten der Zonen in etwa der Größe der Zellen und Poren der natürlichen Spongiosa entsprechen, welche in und an die Oberflächenstruktur des Implantates bzw. durch sie hindurch einwachsen soll.

Wie erwähnt, haben sich die bekannten Implantate in der Praxis hervorragend bewährt. Sie weisen allerdings eine Eigenschaft auf, die sich bei einem Revisionseingriff, d. h. beim Entfernen des Implantates, welches fest mit dem umgebenden Spongiosamaterial verwachsen ist, negativ bemerkbar macht. Die Oberflächenstrukturen der bekannten Implantate sind nämlich so zackig ausgebildet, daß ein Ablösen der in das Material hineingewachsenen Knochentrabekel nicht ohne weiteres möglich ist unter Zuhilfenahme eines geeigneten Werkzeuges, da dieses sich häufig an der zerklüfteten Oberfläche verhakt. Dies stört den Ablauf des Revisionseingriffes in unerwünschter Weise.

Vor diesem Hintergrund ist es die Aufgabe der vorliegenden Erfindung, ein gattungsgemäßes Implantat so weiterzubilden, daß ein Revisionseingriff erleichtert wird und gegenüber dem bekannten Implantat eine noch festere Verankerung des Implantats im Knochenmaterial erzielt wird.

Gelöst wird diese Aufgabe beim gattungsgemäßen Implantat durch das kennzeichnende Merkmal des Anspruchs 1. Vorteilhafte Weiterbildungen sind in den Unteransprüchen angegeben.

Erfindungsgemäß wird also vorgeschlagen, die schon beim gattungsgemäßen Implantat vorhandenen Partikel, welche die offenmaschige dreidimensionale Struktur bilden, anders auszubilden als beispielsweise in der DE-C-41 06 971 angegeben. So wird vorgeschlagen, daß die Enden der zumindest frei von der Basisstruktur abragenden Zapfen Hinterschnitte ausbilden und eine nach außen hin abgerundete Oberfläche aufweisen. Basisstruktur des Implantates kann beispielsweise der massive Kern sein, auf dem die offenmaschige, dreidimensionale Struktur ausgebildet sein soll. Zumindest ragt also ein Zapfen von dieser Basisstruktur weg und weist das speziell ausgebildete Ende auf. Die Enden des bzw. der Zapfen sind nach außen hin abgerundete Materialanlagerungen, welche das Durchtrennen der Knochentrabekel im Falle eines Revisionseingriffes erleichtern, dadurch, daß trotz der Einwachs- und Durchwachseigenschaften der Oberflächenstruktur des Implantates ein Werkzeug relativ leicht über die Oberfläche des Implantats geführt werden kann, ohne daß es zu einem Verhaken mit der Oberflächenstruktur käme. Die Ausbildung der Hinterschnitte durch die abgerundeten Materialanhäufungen ist allerdings auch vor dem zweiten Teil der der Erfindung zugrundeliegenden Aufgabe zu sehen. Durch die Hinterschnitte nämlich wird die Sekundärfixation des Implantates gegenüber dem gattungsgemäßen Implantat noch dadurch erhöht, daß eben die Knochentrapekel, welche durch die offenmaschige und dreidimensionale Oberflächenstruktur hindurchwachsen, noch zusätzlich rein auf mechanische Art und Weise zu der Fixation beitragen, indem praktisch durch eine Art Formenschluß das Knochenmaterial hinter die Hinterschnitte wächst und so zu einer noch höheren Fixationssicherheit beiträgt.

In FR-A-2 356 465 ist ein feingießtechnisches Verfahren zur Herstellung chirurgischer Prothesen beschrieben, bei dem das fertige Gußstück eine im wesentlichen körnige Oberfläche aufweist, die aus einer Vielzahl von Kugeln dargestellt werden, so daß geringfügige Hinterschnitte für das an die Oberfläche des Implantates heranwachsende Knochenmaterial ausgebildet sind.

Gemäß einer bevorzugten Ausführungsform ist vorgesehen, daß die Enden der Zapfen ballig verdickt ausgebildet sind. Die ballige Ausbildung kann bevorzugt kugelförmig oder olivenförmig ausgebildet sein.

Die balligen Verdickungen bilden in diesen Fällen die Hinterschnitte, die für ein mechanisches Zurückhalten des Implantates durch das bereits in die Oberflächenstruktur eingewachsene Knochenmaterial sorgen. Andererseits bewirken die balligen Verdickungen definierte Oberflächenunebenheiten, welche einem Werkzeug bei einem Revisionseingriff beim Durchtrennen der Knochentrabekel nicht hinderlich entgegenstehen.

Eine alternative Ausführungsform zu der balligen Verdickung der Enden der Zapfen ist durch die Ausführungsform gegeben, bei der die Enden der Zapfen pilzförmig ausgebildet sind mit zur Basisstruktur des Implantates gewölbtem Dach. Auch hierdurch läßt sich eine definierte Unebenheit der Oberflächenstruktur erzielen im Hinblick auf einen leichten Revisionseingriff. Allerdings sollte selbstverständlich die Oberfläche nicht praktisch völlig glatt sein, da durch die Unebenheiten der umgebenden Knochen auch zum Wachstum in die Oberflächenstruktur hin angeregt wind.

Unabhängig von der konkreten Ausführung der Enden der Partikelzapfen können die Partikel und die Basisstruktur des Implantates vorzugsweise einstückig aus demselben Material bestehen. Das Material kann körperverträgliches Kunststoff sein, aber auch ein körperverträgliches Metall. Das Implantat wird dann vorzugsweise in einem feingießtechnischen Verfahren hergestellt.

Die Erfindung wird anhand eines Ausführungsbeispiels gemäß der Zeichnungsfiguren näher erläutert: Hierbei zeigt:
- Fig. 1: die schematisierte Ansicht eines Implants in Form eines Hüftstiels,
- Fig. 2: die Vergrößerung der Einzelheit Z in Fig. 2 in zweifacher Vergrößerung, und
- Fig. 3: die Einzelheit Z in Fig. 1 in fünffacher Vergrößerung.

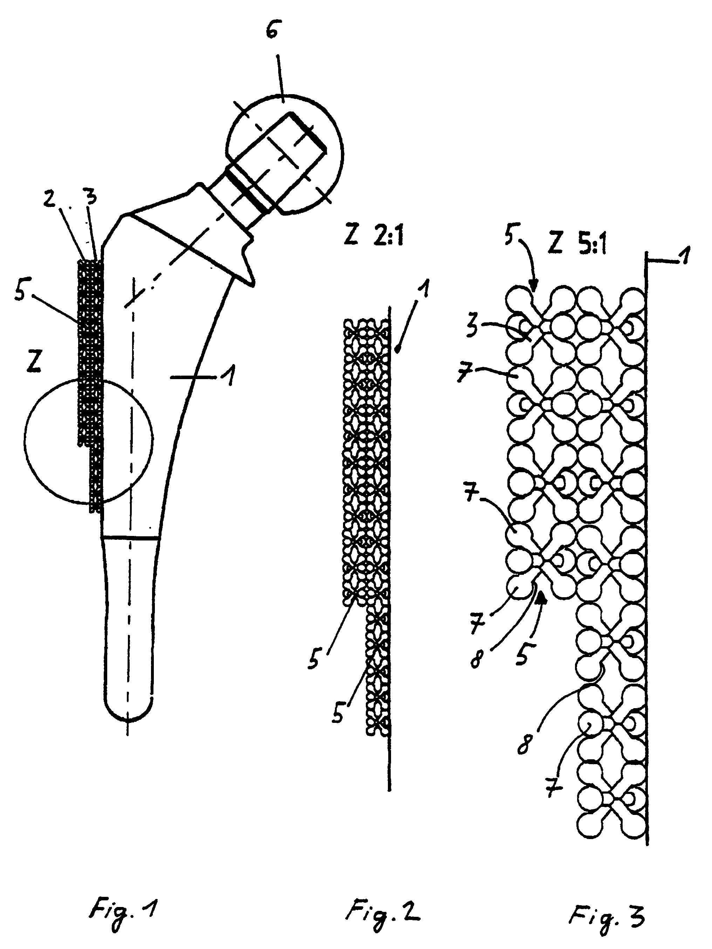

Als Ausführungsbeispiel ist in Fig. 1 ein Implantat in Form eines Hüftstiels gezeigt, welches eine Basisstruktur 1 als massiven Kern sowie einen Kugelgelenkkopf 6 aufweist. Ein Teil der Oberfläche der Basisstruktur 1 ist bedeckt durch eine offenmaschige, dreidimensionale Struktur, die im dargestellten Falle aus zwei Schichten 2 und 3 übereinander geschichteter Partikel 5 besteht. Die Schichten 2 und 3 werden nach Implantation in einen Röhrenknochen, vorliegend also in einen Femurknochen, durchwachsen von Knochentrabekeln der Spongiosa, so daß es zu einem überaus innigen Verbund zwischen dem Implantat und der sie umgebenden Spongiosa kommt.

Aus Fig. 2 wird deutlich, welch enges Maschenwerk durch die Partikel 5 gebildet wird. Durch die Maschen hindurch wächst das Knochenmaterial und sorgt so für eine stabile Sekundärfixation.

Einzelheiten des Aufbaus der Partikel 5 ergeben sich aus der Fig. 3. Diese bestehen vorliegend aus sechs voneinander wegstrebenden Zapfen 3, von denen wenigstens drei mit der Basisstruktur 1 verbunden sind.

Die Enden 7 der Zapfen 3 sind vorliegend ballig verdickt ausgebildet, und zwar kugelförmig. Dies führt zum einen zur Ausbildung von Hinterschnitten 8 hinter der Verdickung hin zum Zapfen 3. Zum anderen wird hierdurch zumindest an der äußeren Schicht von Partikeln eine nach außen hin abgerundete Oberfläche ausgebildet, welche das Führen eines Instrumentes zum Ablösen des in die Schichten 2 und 3 eingewachsenen Knochenmaterials erleichtert. Ein Verhaken des Trenninstrumentes wird bei einer Durchführung eines Revisionseingriffs somit unwahrscheinlich.

Wie bereits erwähnt, führt die Ausbildung der Hinterschnitte 8 zu einer rein mechanisch wirkenden Erhöhung der Sekundärfixation des Implantates in oder an dem Knochen, in welchen bzw. an welchen das Implantat implantiert worden ist.

## Patentansprüche

1. Implantat als Knochenersatz mit einer seine Oberfläche zumindest teilweise bedeckenden offenmaschigen, dreidimensionalen Struktur, die gebildet ist aus Partikeln (5), die mit einer Basisstruktur (1) des Implantats verbunden sind und aus radial voneinander abgehenden vier bis acht Zapfen (3) bestehen, von denen wenigstens drei mit der Basisstruktur (1) verbunden sind,
**dadurch gekennzeichnet,**
**daß** die Enden (7) der zumindest frei von der Basisstruktur (1) abragenden Zapfen (3) Hinterschnitte (8) ausbilden und eine nach außen hin abgerundete Oberfläche aufweisen.

2. Implantat nach Anspruch 1, **dadurch gekennzeichnet, daß** die Enden (7) der Zapfen (3) ballig verdickt ausgebildet sind.

3. Implantat nach Anspruch 2, **dadurch gekennzeichnet, daß** die Enden (7) der Zapfen (3) kugelförmig ausgebildet sind.

4. Implantat nach Anspruch 2, **dadurch gekennzeichnet, daß** die Enden (7) der Zapfen olivenförmig ausgebildet sind.

5. Implantat nach Anspruch 1, **dadurch gekennzeichnet, daß** die Enden (7) der Zapfen (3) pilzförmig ausgebildet sind mit zur Basisstruktur (1) gewölbtem Dach.

6. Implantat nach einem der Ansprüche 1 bis 5, **dadurch gekennzeichnet, daß** die Partikel (5) und die Basisstruktur (1) einstückig aus demselben Material bestehen.

## Claims

1. Implant as bone replacement having an open-meshed, three-dimensional structure at least partly covering its surface, and which is formed from particles (5) which are joined to a base structure (1) of the implant and consist of four to eight pins (3) branching radially from one another, of which at least three are joined to the base structure (1), **characterised in that** the ends (7) of the pins (3) projecting at least free of the base structure (1) form undercuts (8) and have a surface rounded towards the outside.

2. Implant according to claim 1, **characterised in that** the ends (7) of the pins (3) are designed to be thickened spherically.

3. Implant according to claim 2, **characterised in that** the ends (7) of the pins (3) are designed to be spherical.

4. Implant according to claim 2, **characterised in that** the ends (7) of the pins are designed to be olive-shaped.

5. Implant according to claim 1, **characterised in that** the ends (7) of the pins (3) are designed to be mushroom-shaped with a top arched towards the base structure (1).

6. Implant according to one of claims I to 5, **characterised in that** the particles (5) and the base structure (1) integrally consist of the same material.

## Revendications

1. Implant servant de prothèse osseuse avec une structure tridimensionnelle à mailles ouvertes couvrant au moins en partie sa surface, constituée de particules (5), fixées à une structure de base (1) de l'implant, et composée de quatre à huit tiges (3) opposées radialement les unes aux autres, dont trois au moins sont fixées à la structure de base (1),
dans lequel
les extrémités (7) au moins des tiges (3) qui partent librement de la structure de base (1) forment un renfoncement (8) et présentent une surface arrondie vers l'extérieur.

2. Implant selon la revendication 1, dans lequel les extrémités (7) des tiges (3) sont de forme bombée.

3. Implant selon la revendication 2, dans lequel les extrémités (7) des tiges (3) sont de forme ronde.

4. Implant selon la revendication 2, dans lequel les extrémités (7) des tiges (3) sont de forme ovale.

5. Implant selon la revendication 1, dans lequel les extrémités (7) des tiges (3) sont en forme de champignon avec la paroi voûtée tournée vers la structure de base (1).

6. Implant selon l'une quelconque des revendications 1 à 5, dans lequel les particules (5) et la structure de base (1) sont constituées d'une seule pièce d'un même matériau.
